# EUROPÄISCHE PATENTSCHRIFT

(11) **EP 4 166 174 B1**
(45) Veröffentlichungstag und Bekanntmachung des Hinweises auf die Patenterteilung: **12.02.2025**
(21) Anmeldenummer: 21202966.4
(22) Anmeldetag: 15.10.2021
(51) Int. Cl.: A61M 11/00, A61M 15/00, A61M 15/02, B05B 11/00

(54) **FLÜSSIGKEITSSPENDER**
LIQUID DISPENSER
DISTRIBUTEUR DE LIQUIDE

(43) Veröffentlichungstag der Anmeldung: 19.04.2023
(73) Patentinhaber: Aptar Radolfzell GmbH, 78315 Radolfzell (DE)
(72) Erfinder: Fang, Shuting, 94034 Passau (DE)
(74) Vertreter: Witte, Weller und Partner Patentanwälte mbB Stuttgart

(56) Entgegenhaltungen:
- EP-A1- 2 492 019
- WO-A1-2005/044354
- WO-A1-2017/191025
- US-A1- 2002 008 122
- US-B1- 6 244 469

## Beschreibung

### ANWENDUNGSGEBIET UND STAND DER TECHNIK

Die Erfindung betrifft Flüssigkeitsspender zum Austrag von Flüssigkeiten. Es handelt sich primär um Flüssigkeitsspender, die vom Nutzer im Alltag mitgeführt werden und die in einem Flüssigkeitsspeicher ein maximales Flüssigkeitsvolumen von vorzugweise 200 ml oder weniger enthalten.

Insbesondere geht es um das Feld von Flüssigkeitsspendern zur Verwendung mit einer Nikotin oder auch Cannabis enthaltenden Flüssigkeit. Solche Spender werden als Ersatz für Tabakwaren und dergleichen verwendet.

Bei Flüssigkeitsspendern und insbesondere bei den genannten Spendern für Nikotin oder auch Cannabis enthaltende Flüssigkeit ist eine bequeme und einfache Art der Betätigung von großem Wert. Sie gestattet es, einen solchen Flüssigkeitsspender gleichsam beiläufig bspw. aus einer Hosentasche zu entnehmen und durch Betätigung Flüssigkeit oral einzunehmen.

Allerdings ist es bei einem solchen Flüssigkeitsspender weiterhin natürlich gewollt, dass es zu keinem ungewollten Austrag kommt, beispielsweise in der Hosentasche.

Aus der WO 2005/044354 A1 ist ein Flüssigkeitsspender mit einer Kappe zum Schutz einer Austragdüse bekannt. Diese Kappe wirkt mit einem Betätigungshebel derart zusammen, dass im aufgesetzten Zustand der Kappe eine Betätigung des Betätigungshebels nicht möglich ist.

### AUFGABE UND LÖSUNG

Aufgabe der Erfindung ist es insbesondere, einen Flüssigkeitsspender gattungsgemäßer Art zur Verfügung zu stellen, der eine bequeme Art der Bedienung und die Sicherheit gegen ungewollten Austrag von Flüssigkeit bietet.

Erfindungsgemäß wird gemäß der Erfindung ein Flüssigkeitsspender vorgeschlagen, der über einen Flüssigkeitsspeicher zur Lagerung der Flüssigkeit vor dem Austrag und über eine Düseneinheit verfügt, durch die Flüssigkeit austragbar ist. Um Flüssigkeit auszutragen, kann eine mittels einer Betätigungshandhabe betätigbare Pumpeinrichtung vorgesehen sein, mittels derer Flüssigkeit aus dem nicht unter Druck stehenden Flüssigkeitsspeicher gefördert wird. Alternativ kann die Betätigungshandhabe auch mit einem Auslassventil wirkverbunden sein, so dass durch Betätigung der Betätigungshandhabe das Auslassventil geöffnet wird und Flüssigkeit aus einem in diesem Falle als Druckspeicher ausgebildeten Flüssigkeitsspeicher zur Düseneinheit gefördert wird. Die Düseneinheit ist im einfachsten Falle durch eine einzelne Austragöffnung gebildet. Bevorzugt ist die Düse jedoch zur Erzeugung eines Sprühstoßes ausgebildet, beispielsweise durch eine Austragöffnung, der eine Wirbelkammer vorgeschaltet ist, oder durch eine Düsenplatte mit einer Mehrzahl von Düsenöffnungen, die parallele, divergierende oder konvergierende Einzelstrahlen erzeugt. Insbesondere erzeugen die Düsenöffnungen Einzelstrahlen, die ausreichend fein sind, um mittels Rayleigh-Zerfall unabhängig vom Lufteinfluss in Einzeltropfen zu zerfallen.

Gemäß der Erfindung ist vorgesehen, dass der Flüssigkeitsspender ein Innengehäuse aufweist. Dieses Innengehäuse ist ein Gehäuseteil, an dem die Betätigungshandhabe in schwenkbarer Form angebracht ist, insbesondere mittels Schwenkzapfen an einem der Teile, vorzugsweise an der Betätigungshandhabe, und korrespondierenden Aufnahmelöchern an dem anderen Teil, vorzugsweise dem Innengehäuse. Weiterhin weist das Innengehäuse vorzugsweise eine Durchbrechung auf, die der mittelbaren Aufnahme der genannten Düse und insbesondere der genannten Düsenplatte dient. Das Innengehäuse kann bei einer bevorzugten Ausgestaltung von außen nicht oder nur zu geringen Anteilen erkennbar sein, da es von anderen Gehäuseteilen umgeben ist. Es kann hierdurch in vorteilhafter Weise eine alleine unter technischen Gesichtspunkten gestaltete Formgebung aufweisen.

Zusätzlich zum Innengehäuse ist ein Außengehäuse vorgesehen. Dieses bildet vorzugsweise zusammen mit der Betätigungshandhabe einen umlaufend umgebenen Innenraum, in dem das Innengehäuse angeordnet ist. Vorzugsweise bilden die Betätigungshandhabe und das Außengehäuse den überwiegenden Teil einer Außenkontur des Flüssigkeitsspenders. Das Außengehäuse ist gegenüber dem Innengehäuse und damit auch gegenüber der daran angebrachten Betätigungshandhabe translativ zwischen einer ersten Endlage und einer zweiten Endlage verschieblich.

Diese Verschiebbarkeit wird genutzt, um einen gesperrten und einen freigegebenen Zustand des Flüssigkeitsspenders erzielen zu können. Zu diesem Zweck weisen die Betätigungshandhabe und das Außengehäuse zusammenwirkende Sperrabschnitte auf. In der ersten Endlage des Außengehäuses zum Innengehäuse unterbinden die Sperrabschnitte eine Betätigung der Betätigungshandhabe. In der zweiten Endlage ist dagegen die Schwenkbetätigung der Betätigungshandhabe ermöglicht.

Die Sperrabschnitte sind vorzugsweise einstückig mit benachbarten Wandungen des Außengehäuses bzw. des Innengehäuses ausgebildete Strukturen, insbesondere Erhebungen oder Ausnehmungen, die im gesperrten Zustand bei einer Schwenkbewegung der Betätigungshandhabe kollidieren und dadurch den Austrag verhindern. Die Betätigungshandhabe kann im gesperrten Zustand nicht ausreichend weit verschwenkt werden, um eine Pumpeinrichtung zu betätigen oder ein Auslassventil zu öffnen. Im freigegebenen Zustand sind diese Sperrstrukturen entsprechend der erfolgten translativen Verlagerung von Innengehäuse und Außengehäuse in einer versetzten Stellung, in der sie das Verschwenken der Betätigungshandhabe gestatten.

Das Erfordernis, ausgehend vom gesperrten Zustand zunächst eine translative Verlagerung des Innengehäuses gegenüber dem Außengehäuse bewirken zu können, stellt einen wirksamen Schutz gegen eine unbeabsichtigte Betätigung dar. Der Nutzer kann den Flüssigkeitsspender im gesperrten Zustand in einer Tasche mit sich führen und ihn beim Herausholen mit einem Handgriff in den freigegebenen Zustand überführen.

Allerdings ist es von Vorteil, wenn konstruktiv gewährleistet ist, dass die translative Verlagerung nicht unbeabsichtigt stattfindet. Um dies zu erreichen, können das Innengehäuse und das Außengehäuse reibungsbehaftet aneinander geführt sein.

Demgegenüber von Vorteil ist es jedoch, wenn der Flüssigkeitsspender über eine Blockiereinrichtung verfügt, mittels derer die Verschiebbarkeit des Außengehäuses gegenüber dem Innengehäuse blockierbar ist. Eine solche Blockiereinrichtung macht es bei der Inbetriebnahme des Flüssigkeitsspenders erforderlich, dass zunächst ein separater Handhabungsschritt durchgeführt wird, der nachfolgend die translative Verlagerung von Innengehäuse und Außengehäuse erst gestattet.

Eine bevorzugte Möglichkeit besteht darin, dass am Innengehäuse oder am Außengehäuse ein elastisch auslenkbarer Blockierkörper vorgesehen ist, der in einer nicht oder nur geringfügig ausgelenkten Stellung in einer Blockierausnehmung des Außengehäuses oder des Innengehäuses angeordnet ist und hierdurch die Blockierung bewirkt. Dieser Blockierkörper ist vorzugsweise mittels mindestens einer Freigabetaste derart auslenkbar, dass er aus der Blockierausnehmung ausgerückt wird und nachfolgend die translative Verschiebung zulässt. Der Blockierkörper und die Freigabetaste können beide am Außengehäuse vorgesehen sein, währen die Blockierausnehmung am Innenkörper vorgesehen ist. Bevorzugt wird es allerdings, wenn die Blockierausnehmung und die Freigabetaste am Außenkörper vorgesehen sind, während der Blockierkörper am Innenkörper vorgesehen ist, der gegenüber umgebenden Wandungsteilen des Innenkörpers elastisch auslenkbar ist.

Bevorzugt ist es weiterhin, wenn die Freigabetaste der Betätigungshandhabe gegenüberliegend am Spender vorgesehen ist. Der Flüssigkeitsspender kann dann umgriffen werden, so dass der Daumen auf der Betätigungstaste aufliegt und einer der anderen Finger zum Eindrücken der Freigabetaste genutzt wird. Alternativ kann der Daumen zum Eindrücken der Freigabetaste aufliegen, während die anderen Finger auf der Betätigungshandhabe aufliegen.

Eine Blockierausnehmung am Außengehäuse kann eine Doppelfunktion erfüllen, sofern sie eine Durchbrechung bildet, durch die hindurch eine Indikatorfläche des Innengehäuses von außen sichtbar ist, wenn sich das Innengehäuse und das Außengehäuse in der ersten bzw. der zweiten Endlage befinden. Durch die Durchbrechung hindurch sind die erste und die zweite Endlage einfach erfassbar, wenn in der ersten Endlage eine beispielsweise rote Teilfläche des Innenkörpers erkennbar ist und/oder in der zweiten Endlage eine beispielsweise grüne Teilfläche. Eine solche Gestaltung mit Indikatorfläche und Durchbrechung ist jedoch auch losgelöst von der Blockiereinrichtung und ihrer Blockierausnehmung möglich.

Wenn die Düsenöffnung bzw. die Düsenplatte am Innenkörper oder zu diesem ortsfest ausgebildet ist, wird sie mit diesem translativ bewegt, wenn der Innenkörper gegenüber dem Außenkörper zwischen dem gesperrten und dem freigegebenen Zustand verlagert wird. Dies gestattet es, die Düse in eine durch das Außengehäuse geschützte Lage zu verbringen, wenn sich der Flüssigkeitsspender im gesperrten Zustand befindet.

Insbesondere kann vorgesehen sein, dass die Düseneinheit und/oder ein die Düseneinheit umgebender Austragstutzen derart angeordnet ist, dass die Düseneinheit und/oder der Austragstutzen in der ersten Endlage und somit im gesperrten Spenderzustand innerhalb des Außengehäuses angeordnet ist und in der zweiten Endlage durch eine Öffnung des Außengehäuses hindurch nach außen verlagert wird.

In Hinblick auf die oben bereits beschriebenen Sperrabschnitte, die im gesperrten Zustand die Schwenkbeweglichkeit der Betätigungshandhabe limitieren, werden die folgenden Bauweisen für besonders zweckmäßig gehalten.

Bei einer Bauweise weist das Außengehäuse mindestens eine sich nach innen oder vorzugsweise nach außen erstreckende Sperrkontur auf, vorzugsweise in Art eines Stiftes oder zwei einander gegenüberliegender Stifte. Die Betätigungshandhabe weist korrespondierend mindestens eine in Richtung der mindestens einen Sperrkontur weisende Sperrkante auf, zu der benachbart ein Freibereich angeordnet ist. Wenn das Außengehäuse und das Innengehäuse in der ersten Endlage und somit im gesperrten Zustand sind, so kollidiert die Sperrkante mit der Sperrkontur. In der zweiten Endlage hingegen sind die Sperrkonturen, insbesondere die Stifte, versetzt, so dass sie bei Betätigung der Betätigungshandhabe in den Freibereich gelangen und der zum Flüssigkeitsaustrag erforderlichen Verlagerung nicht mehr entgegenstehen.

Bei einer anderen Bauweise ist vorgesehen, dass das Außengehäuse eine Durchbrechung aufweist, durch die hindurch sich die Betätigungshandhabe von einem Innenbereich des Außengehäuses nach außen erstreckt. Bei einer solchen Struktur ist es möglich, die Betätigungshandhabe derart an die Durchbrechung anzupassen, dass in der ersten Endlage ein distales Ende der Betätigungshandhabe bei Betätigung an einem Randbereich der Durchbrechung anschlägt, so dass ein Flüssigkeitsaustrag verhindert wird. In der zweiten Endlage und somit im freigegebenen Zustand des Spenders ist der genannte Randbereich gegenüber der Betätigungshandhabe verlagert, so dass das distale Ende der Betätigungshandhabe durch die Durchbrechung hindurch in den Innenbereich des Außengehäuses hinein schwenken kann.

Die Düseneinheit ist vorzugsweise mit einer Düsenplatte mit einer Vielzahl von Düsenöffnungen ausgestattet, die zur Erzeugung feiner Sprühstrahlen ausgebildet sind, die unabhängig vom Lufteinfluss nach dem Austritt in Einzeltropfen zerfallen (Rayleigh-Zerstäubung). Vorzugsweise weist die Düsenplatte hierfür mindestens 10 Düsenöffnungen auf. Die Düsenöffnungen weisen vorzugsweise einen lichten Querschnitt von maximal 250 µm² auf.

Um einen ausreichenden Flüssigkeitsstrom beim Austrag dieser feinen Sprühstrahlen zu erzeugen, muss bei Gestaltungen mit einer Pumpeinrichtung ein vergleichsweise hoher Druck erzeugt werden. Um diesen bequem erzeugen zu können, ist als schwenkbewegliche Betätigungshandhabe ausgebildet und weist eine Betätigungsfläche von mindesten 5 cm Länge auf, vorzugsweise von mindestens 6 oder gar mindestens 8 cm Länge auf. Vorzugsweise beträgt auch der Abstand von der Schwenkachse bis zum distalen Ende der Betätigungshandhabe mindesten 5 cm Länge, vorzugsweise von mindestens 6 oder mindestens 8 cm Länge.

Der Benutzer kann daher mit zwei oder mehr Fingern auf die Betätigungshandhabe drücken, wenn er das Außengehäuse mit seiner Hand umschlossen hat. Entsprechend gut kann auch über einen längeren Zeitraum eine hohe Kraft auf die Pumpeinrichtung ausgeübt werden.

Es wird als bevorzugt angesehen, wenn die Betätigungshandhabe an einem Innengehäuse schwenkbar angelenkt ist, gegenüber dem das Außengehäuse translativ verlagerbar ist.

Die Pumpeinrichtung eines erfindungsgemäßen Flüssigkeitsspenders ist vorzugsweise derart ausgelegt, dass ein zur Erzeugung eines feinen Aerosols geeigneter Druck mit der Betätigungshandhabe über einen längeren Zeitraum erzeugt werden kann. Insbesondere ist die Pumpeinrichtung dafür ausgebildet, eine Flüssigkeitsmenge von mindestens 25 µl je Betätigung zu fördern.

Vorzugsweise ist die Pumpeinrichtung derart ausgelegt und mit der Betätigungshandhabe gekoppelt und die Düseneinheit sowie ein Fluidkanal von der Pumpeinrichtung zur Düseneinheit sind vorzugsweise derart ausgelegt, dass ausgehend von einer vollständig mit Wasser befüllten Pumpkammer der Pumpeinrichtung und bei Betätigung der Betätigungshandhabe mit einer Betätigungskraft von 20 Newton am distalen Ende der Betätigungshandhabe ein Austrag von mindestens 1 Sekunden bewirkt wird, insbesondere von mindestens 2 Sekunden. Der Austrag erfolgt dabei zeitgleich mit einer ebenso langen Betätigung der Betätigungshandhabe.

Das Innengehäuse des Flüssigkeitsspenders kann unmittelbar den darin unlösbar verbauten Flüssigkeitsspeicher sowie alle weiteren flüssigkeitsführenden Oberflächen des Spenders umfassen. Insbesondere kann eine Außenwandung des Innengehäuses gleichzeitig die Wandung des Flüssigkeitsspeichers darstellen.

Eine Alternative hierzu sieht vor, dass der Flüssigkeitsspender eine separate Austragbaugruppe aufweist, die mindestens den Flüssigkeitsspeicher, die Pumpeinrichtung oder ein Auslassventil sowie eine Abgabeöffnung umfasst. Die Austragbaugruppe stellt vorzugweise bereits getrennt von anderen Teilen des Flüssigkeitsspenders einen eigenen vollständig funktionsfähigen Spender dar. Dies ermöglicht es, einen bestehenden und hierfür nicht oder nicht weitgehend angepassten Spender zu verwenden. Der konstruktive Aufwand des erfindungsgemäßen Flüssigkeitsspenders wird hierdurch verringert. Ein solcher funktionsfähiger Spender, der als Austragbaugruppe Verwendung findet, ist vorzugsweise ein so genannter "Side Actuation"-Spender, der eine Betätigungstaste im Bereich einer Mantelfläche aufweist und der vorzugsweise über eine quer eingebaute Pumpeinrichtung verfügt.

Die Austragbaugruppe ist dafür vorgesehen, in das Innengehäuse eingesetzt zu werden. Dieses verfügt zu diesem Zweck über einen Aufnahmeraum, in dem die Austragbaugruppe werkzeuglos angekoppelt und werkzeugslos hiervon wieder gelöst werden kann. Das Innengehäuse umgibt diesen Aufnahmeraum vorzugweise vollständig und ist an einer Unterseite offen, damit die Austragbaugruppe hier eingeschoben werden kann.

Die Austragbaugruppe kann im Innengehäuse beispielsweise kraftschlüssig gehalten werden. Demgegenüber bevorzugt sind jedoch Ausgestaltungen, bei denen das Innengehäuse und die Austragbaugruppe zur formschlüssigen Kopplung vorgesehen sind, insbesondere mittels einer Bajonettkupplungseinrichtung oder einer Gewindekopplungseinrichtung.

Bevorzugt ist es, wenn der Flüssigkeitsspender über eine Pumpeinrichtung verfügt, vorzugsweise über eine Pumpeinrichtung mit einer Pumpkammer, die über einen Einlasskanal mit dem Flüssigkeitsspeicher verbunden ist und die über einen Auslasskanal mit der Düseneinheit verbunden ist, wobei der Einlasskanal und der Auslasskanal jeweils mit einem bei stromabwärtigen Unterdruck öffnenden Einlassventil bzw. Auslassventil versehen sind. Vorzugsweise ist die Pumpkammer über einen in einer Pumprichtung verlagerbaren Kolben oder eine Membranwandung volumetrisch veränderlich. Die Pumprichtung ist vorzugsweise nicht-parallel zur Austragrichtung ausgerichtet. Vorzugsweise in die Pumprichtung der Pumpeinrichtung quer zur Austragrichtung ausgerichtet.

Eine Gestaltung des Flüssigkeitsspenders mit einer Pumpeinrichtung gestattet es, einen drucklosen Flüssigkeitsspeicher zu verwenden. Vorzugsweise weist der Flüssigkeitsspeicher ein veränderliches Innenvolumen auf, so dass auf eine Belüftung des Flüssigkeitsspeichers verzichtet werden kann. Insbesondere kann der Flüssigkeitsspeicher eine zylindrische Wandung aufweisen, in die ein verschieblicher Schleppkolben angeordnet ist. Alternativ kann ein Flüssigkeitsspeicher mit mindestens einer auslenkbaren flexiblen Wandung genutzt werden, insbesondere in Form eines kollabierenden Beutelspeichers.

Der Flüssigkeitsspender kann als ganzes als Einwegspender ausgebildet sein. Alternativ kann vorgesehen sein, dass zumindest Teile wiederverwendet werden, indem der Flüssigkeitsspeicher oder die oben beschriebene Austragbaugruppe wechselbar gestaltet sind. Eine weitere Möglichkeit zur Wiederverwendung des Spenders sieht vor, dass der Flüssigkeitsspender eine von der Düseneinheit getrennte Nachfüllöffnung aufweist. Durch diese Nachfüllöffnung kann der Flüssigkeitsspeicher aufgefüllt werden, insbesondere wenn es sich um einen drucklosen Speicher handelt. Die Nachfüllöffnung ist vorzugweise unmittelbar in einer Wandung des Flüssigkeitsspeichers angeordnet.

Im verkaufsfertigen Zustand ist der Flüssigkeitsspeicher befüllt, wobei er entsprechend der bevorzugten Anwendungsfelder vorzugsweise mit einer pharmazeutischen Flüssigkeit oder mit einer nikotinhaltigen und/oder cannabishaltigen Flüssigkeit befüllt ist. Insbesondere kann ein Flüssigkeitsvolumen von 50 ml oder weniger, insbesondere von 10 ml oder weniger, im Flüssigkeitsspeicher enthalten sein.

Die Düseneinheit, die vorzugsweise eine Düsenplatte und einen die Düsenplatte tragenden Träger aufweist, kann als werkzeuglos wechselbare Düseneinheit ausgebildet sein, beispielsweise mit dem Hauptkörper des Innengehäuses mittels einer Bajonettkupplung verbindbar und hiervon lösbar. Eine wechselbare Düseneinheit kann zum Zwecke der Reinigung einfach entnommen werden und kann darüber hinaus der optischen Individualisierung des Flüssigkeitsspenders dienen.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 bis 10 zeigen ein erstes Ausführungsbeispiel eines erfindungsgemäßen Flüssigkeitsspenders.
Fig. 1 und 2 zeigen den Spender im freigegebenen Zustand in einer perspektivischen Darstellung und einer Schnittdarstellung.
Fig. 3 zeigt die Düseneinheit des Spenders mit einem umgebenden Träger.
Fig. 4 bis 7 verdeutlichen das Lösen einer Blockiereinrichtung des Spenders und das nachfolgende Überführen des Flüssigkeitsspenders von einem gesperrten Zustand in einen freigegebenen Zustand.
Fig. 8 verdeutlicht den Aufbau des Flüssigkeitsspenders durch getrennte Darstellung seines Außengehäuses, seines Innengehäuses mitsamt Betätigungshandhabe sowie seiner Austragbaugruppe.
Fig. 9 zeigt den Verbund von Innengehäuse und Außengehäuse vor dem Einsetzen der Austragbaugruppe.
Fig. 10 zeigt das Einsetzen des Austragbaugruppe in den Verbund der Fig. 9.
Fig. 11 bis 13 zeigen ein zweites Ausführungsbeispiel eines erfindungsgemäßen Flüssigkeitsspenders mit abweichender Gestaltung der Sperre zur Verhinderung von Flüssigkeitsaustrag im gesperrten Zustand des Spenders.
Fig. 14 und 15 zeigen eine Variante des Flüssigkeitsspeichers des Flüssigkeitsspenders in Ausgestaltung als Flüssigkeitsbeutel.
Fig. 16 zeigt eine Variante des Flüssigkeitsspeichers des Flüssigkeitsspenders mit einem Schleppkolben.
Fig. 17 und 18 zeigen eine Variante des Flüssigkeitsspenders, bei der die Düseneinheit werkzeuglos wechselbar gestaltet ist.

### DETAILLIERTE BESCHREIBUNG DER AUSFÜHRUNGSBEISPIELE

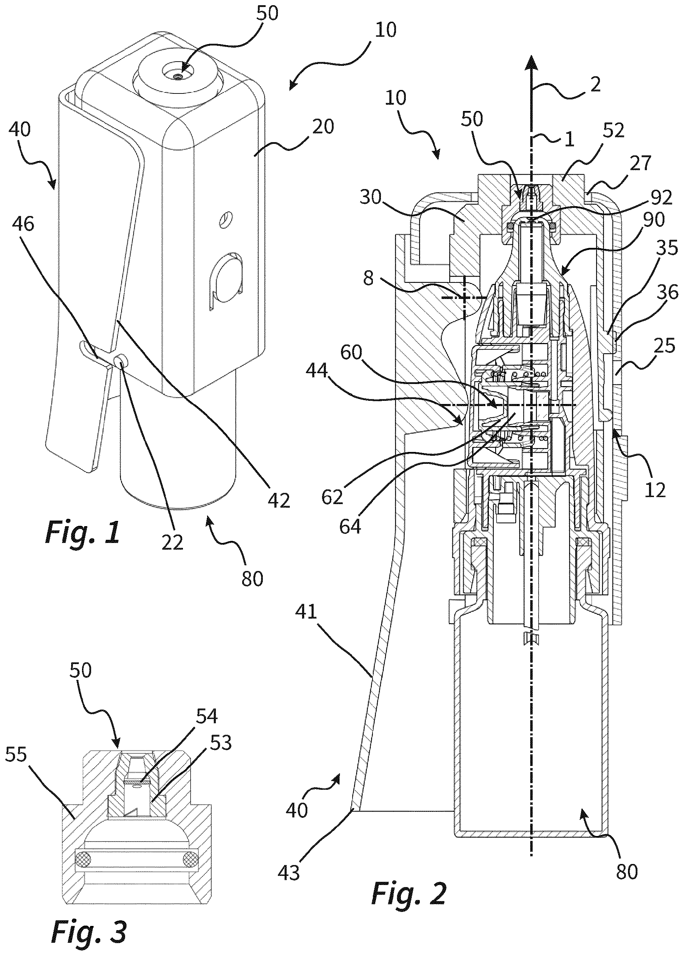

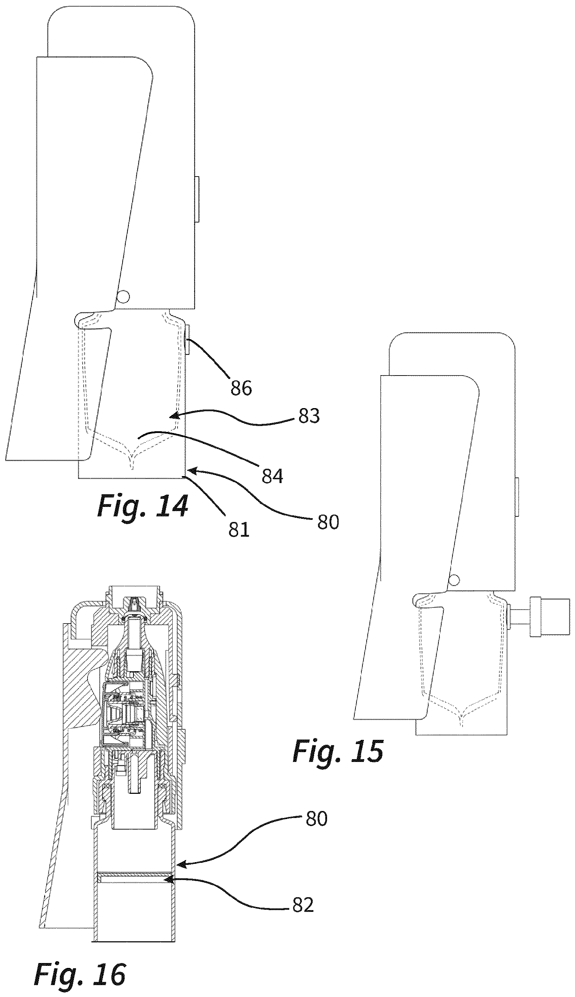

Die Gestaltungen der Fig. 1 bis 10 und der Fig. 11 bis 13 unterscheiden sich durch bauliche Details, die im Weiteren noch erläutert werden. Soweit nicht anders erläutert, gelten die nachfolgend beschriebenen Aspekte für beide Gestaltungen. Die weiteren baulichen Details, die anhand der Figuren 14 bis 18 erläutert sind, können gleichermaßen bei Flüssigkeitsspendern der Fig. 1 bis 10 oder 11 bis 13 realisiert sein.

Die Fig. 1 und 2 zeigen einen Flüssigkeitsspender erfindungsgemäßer Art in einer perspektivischen Darstellung sowie einer geschnittenen Darstellung. Der Flüssigkeitsspender 10 verfügt über ein Außengehäuse 20, ein hierein eingesetztes Innengehäuse 30 mit einer darin enthaltenen Austragbaugruppe 90 sowie über eine Betätigungshandhabe 40. Der Flüssigkeitsspender 10 weist insgesamt eine in Richtung einer Hauptachse 1 länglich ausgerichtete Formgebung auf. An dem in den Figuren nach oben weisenden Ende des Flüssigkeitsspenders 10 ist eine Düseneinheit 50 vorgesehen, durch die Flüssigkeit in einer Austragrichtung 2 ausgetragen werden kann, insbesondere in fein zerstäubter Form.

Zur Bewirkung eines Flüssigkeitsaustrags ist die Betätigungshandhabe 40 vorgesehen, die um eine zum Innengehäuse 30 ortsfeste Schwenkachse 8 schwenkbar ist und welche hierdurch in noch erläuterter Weise eine Pumpeinrichtung 60 betätigen kann, mittels derer Flüssigkeit aus einem Flüssigkeitsspeicher 80 zur Düseneinheit 50 gefördert werden kann. Die Pumpeinrichtung 60 ist beim vorliegenden Ausführungsbeispiel als Membrankolbenpumpe ausgebildet. Ihre Pumpkammer 62 kann durch Verlagerung des Membrankolbens 64 orthogonal zur Hauptachse 1 verkleinert und vergrößert werden. Der Membrankolben 64 weist einen vergleichsweise starren zentrischen Teil auf, der über eine verformbare Ringfläche randseitig mit einer festen Pumpkammerwandung verbunden ist.

In der Pumpkammer62 befindliche Flüssigkeit wird bei Betätigung der Betätigungshandhabe 40 und hieraus resultierender Verlagerung des Membrankolbens 64 gefördert und hier eine in Fig. 3 dargestellte Düsenplatte 54 der Düseneinheit 50 hindurchgedrückt. Die Düsenplatte 54 weist eine Vielzahl feiner Düsenöffnungen auf, die beispielsweise mittels eines Lasers in die Düsenplatte eingebracht worden sein können. Die beispielsweise metallische Düsenplatte 54 ist in eine Düsenhülse 53 eingesetzt.

Es ist insbesondere gewünscht, dass die durch die Düsenplatte 54 abgegebenen feinen Flüssigkeitsstrahlen nach Abgabe in feinste Einzeltröpfchen zerfallen. Bei geeigneter Auslegung der Düsenöffnungen der Düsenplatte 54 sind die Flüssigkeitsstrahlen ausreichend fein, um einen sogenannten Rayleigh-Zerfalljenseits der Düsenplatte zu gewährleisten.

Die Düsenplatte weist hierfür 50 Düsenöffnungen auf, deren lichter Querschnitt an der engsten Stelle jeweils 200 µm² beträgt.

Die Düsenplatte 54 ist an die Pumpeinrichtung 60, den durch diese erzeugbaren Druck und die durch eine Betätigung der Pumpeinrichtung 60 austragbare Flüssigkeitsmenge derart angepasst, dass im Zuge einer einzelnen Pumpbewegung durch Betätigung mit 20 Newton für einen Zeitraum von mindestens eine Sekunden Flüssigkeit derart durch die Düsenplatte 54 austragbar ist, dass die entstehenden Flüssigkeitsstrahlen in der gewünschten Weise in Einzeltröpfchen zerfallen.

Die schwenkbewegliche Betätigungshandhabe 40, die über eine Nocke 44 mittelbar auf den Membrankolben 64 wirkt, gestattet eine lang anhaltende und hierbei recht bequeme Betätigung. Dies wird insbesondere dadurch erzielt, dass eine große Betätigungsfläche 41 vorgesehen ist. Der Abstand zwischen der Schwenkachse 8 der Betätigungshandhabe 40 und ihrem distalen Ende 43 beträgt im vorliegenden Fall etwa 7 cm. Da die Nocke 44 nur knapp 2 cm von der Schwenkachse 8 entfernt ist, kann mit einer Betätigungskraft von 20 Newton am distalen Ende 43 der Betätigungshandhabe 40 in etwa eine Kraft von 80 Newton auf den Membrankolben 64 ausgeübt werden.

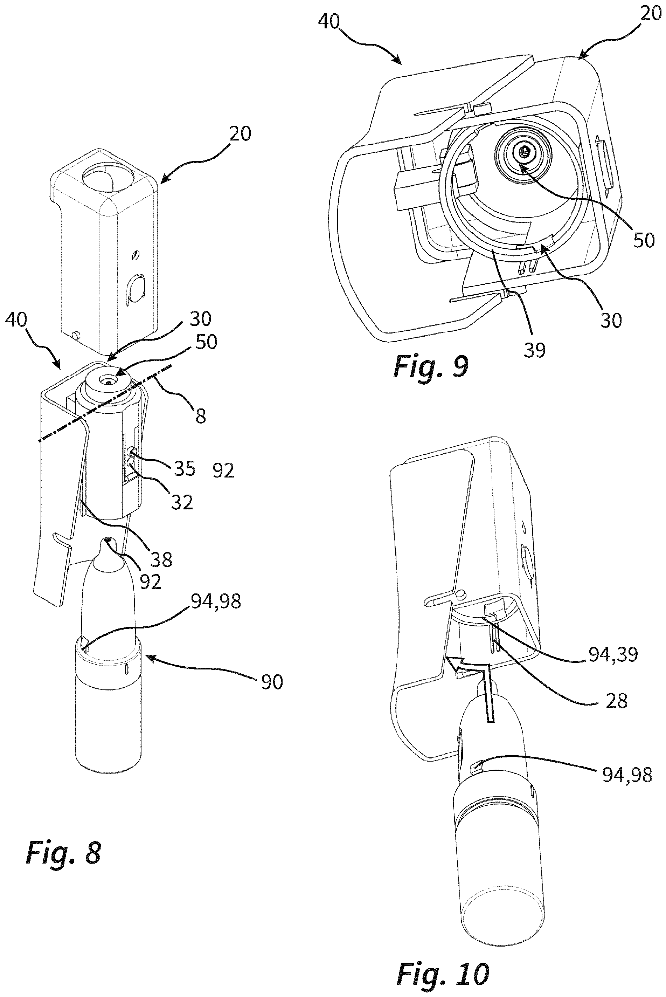

Die Pumpeinrichtung 60 ist Teil einer Austragbaugruppe 90, die darüber hinaus auch den Flüssigkeitsspeicher 80 sowie eine Abgabeöffnung 92 aufweist. Die in Fig. 8 und 10 nochmals separat dargestellte Austragbaugruppe 90 ist dadurch für sich genommen bereits ein funktionstauglicher Spender zum Austrag von Flüssigkeit, der durch Ergänzung um das Innengehäuse 30, das Außengehäuse 20 sowie die Betätigungshandhabe 40 funktional ergänzt wird.

In der bereits erwähnten Fig. 3 ist die Düseneinheit 50 dargestellt. Diese ist in einen Träger 55 eingesetzt, der zusätzlich an seiner Unterseite einen Aufnahmeraum für das distale Ende der Austragbaugruppe 90 aufweist. Dies bedeutet, dass Flüssigkeit, die aus der Austragbaugruppe im Bereich der Abgabeöffnung 92 abgegeben wird, sich in der mittels eines O-Rings abgedichteten Ausnehmung des Trägers 55 sammelt und von hier aus durch die Düsenplatte 54 in Form der genannten Flüssigkeitsstrahlen abgegeben wird.

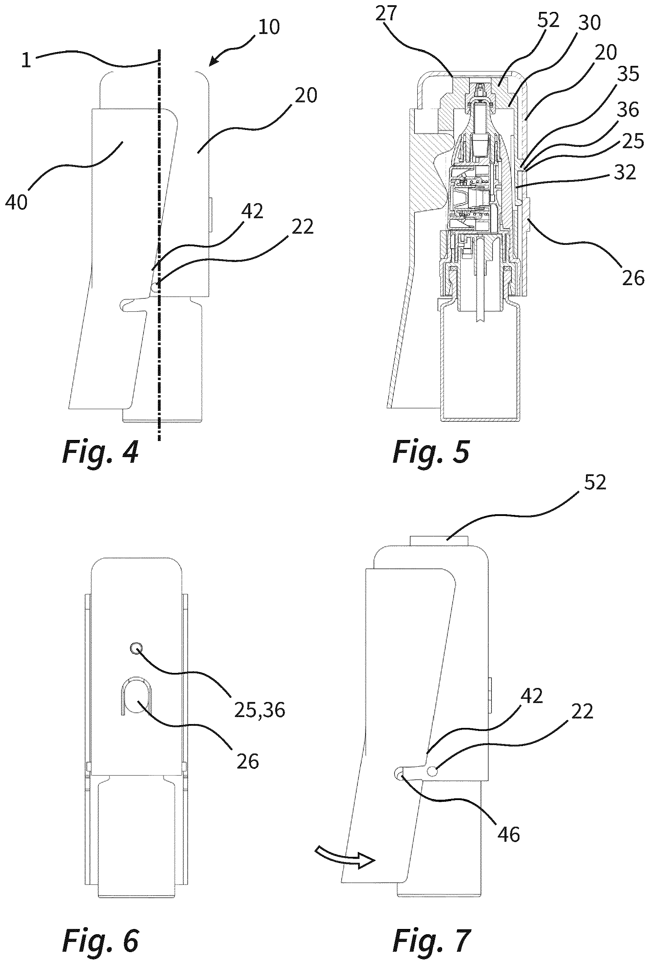

Die Fig. 1 und 2 zeigen einen entsperrten Zustand des Flüssigkeitsspenders 10. Der Flüssigkeitsspender 10 kann jedoch neben dem entsperrten Zustand, aus dem heraus eine Flüssigkeitsabgabe möglich ist, auch einen gesperrten Zustand einnehmen. Dies ist in den Fig. 4 und 5 dargestellt. In dem gesperrten Zustand der Fig. 4 und 5 ist das Außengehäuse 20 gegenüber allen anderen Komponenten des Flüssigkeitsspenders 10, so insbesondere gegenüber dem Innengehäuse 30, der Betätigungshandhabe 40 sowie der im Innengehäuse 30 befestigten Austragbaugruppe 90 in Richtung der durch die Haupterstreckungsrichtung definierte Hauptachse 1 nach oben verschoben. In diesem Zustand ist eine Betätigung der Pumpeinrichtung 60 nicht möglich, da die Betätigungshandhabe 40 mit einer Sperrkante 42 bei Betätigung in Kontakt mit einem Sperrstift 22 gelangt, der eine fortgesetzte Betätigung der Betätigungshandhabe 40 unterbindet. Dieser gesperrte Zustand der Fig. 4 und 5 ist dadurch gesichert, dass in einer Blockierausnehmung 25 des Außengehäuses 20 ein Blockierkörper 35 angeordnet ist, der einer axialen Verlagerung des Außengehäuses 20 gegenüber dem Innengehäuse 30 entgegenwirkt.

Um ausgehend vom Zustand der Fig. 4 und 5 den freigegebenen Zustand der Fig. 1 und 2 herzustellen, hat der Benutzer auf eine Freigabetaste 26 zu drücken. Diese Freigabetaste 26, die einstückiger Teil des Außengehäuses 20 ist, gegenüber den umgebenden Wandungsbereichen des Außengehäuses 20 jedoch elastisch auslenkbar ist, wirkt auf einen ebenfalls elastisch auslenkbaren Abschnitt 32 des Innengehäuses 30, wodurch der Blockierkörper 35 aus der Blockierausnehmung 25 nach innen herausgehoben wird, so dass anschließend eine axiale Verlagerung des Außengehäuses 20 gegenüber dem Innengehäuse 30 möglich ist. Solange jedoch der Flüssigkeitsspender 10 sich im gesperrten Zustand befindet, ist eine Indikatorfläche 36, die vorzugsweise mit der Signalfarbe Rot versehen ist, durch die Durchbrechung25 hindurch von außen erkennbar, so dass mit einem Blick erfasst werden kann, dass der Flüssigkeitsspender sich derzeit im gesperrten Zustand befindet.

Wenn durch Eindrücken der Freigabetaste 26 und nachfolgende Relativverschiebung des Außengehäuses 20 gegenüber dem Innengehäuse 30, der Betätigungshandhabe 40 und der Austragbaugruppe 90 der freigegebene Zustand hergestellt ist, so stellt sich dieser entsprechend der Fig. 7 dar.

Es ist hier zu ersehen, dass nunmehr die Sperrkante 42 gegenüber dem Sperrstift 22 verlagert ist, so dass der Sperrstift 22 bei Betätigung der Betätigungshandhabe entsprechend dem in Fig. 7 eingezeichneten Pfeil in einen Freibereich 46 die Betätigungshandhabe 40 eintauchen kann, so dass die Pumpeinrichtung 60 betätigt werden kann.

Wie beim Vergleich der Fig. 7 und 4 gut erkennbar ist, führt die translative Verlagerung des Außengehäuses 20 gegenüber dem Innengehäuse 30 auch dazu, dass im freigegebenen Zustand der Fig. 7 ein die Düseneinheit 50 umgebender Austragstutzen 52 des Innengehäuses 30 durch eine korrespondierende Öffnung 27 des Außengehäuses 20 nach außen geschoben ist. Im gesperrten Zustand der Fig. 4 dagegen ist der Austragstutzen und mit ihm die darin eingesetzte Düseneinheit 50 vollständig innerhalb des Außengehäuses 20 angeordnet und dadurch vor mechanischen Beschädigungen geschützt.

Die Fig. 8 zeigt nochmals den Flüssigkeitsspender 10, aufgegliedert nach Teilkomponenten. Zu erkennen ist die Austragbaugruppe 90, die durch das Innengehäuse 30 und das Außengehäuse 20 zu einem erfindungsgemäßen Flüssigkeitsspender ergänzt ist, jedoch auch ohne diese weiteren Elemente 20, 30, 40 einen funktionstauglichen Spender aufweist. Zu erkennen ist weiterhin das Innengehäuse 30 mit der daran um die Schwenkachse 8 schwenkbaren Betätigungshandhabe 40. Wie hier zu ersehen ist, weist das Innengehäuse 30 eine grundsätzlich hülsenförmige Struktur auf. An der in Fig. 8 nach vorne weisenden Seite ist der hier elastisch auslenkbare Blockierkörper 35 vorgesehen. Zu erkennen ist weiterhin, dass seitlich am Innengehäuse 30 Führungsstege 38 vorgesehen sind, die mit in Fig. 10 dargestellten Nuten 28 zusammenwirken, um die translativ geführte Beweglichkeit des Außengehäuses 20 gegenüber dem Innengehäuse 30 und der Betätigungshandhabe 40 zu gestatten.

Fig. 9 zeigt den Verbund aus dem Innengehäuse 30, dem Außengehäuse 20 und der Betätigungshandhabe 40. Es ist zu ersehen, dass das Innengehäuse 30 über eine Bajonettnut 39 verfügt, die in der in Fig. 10 verdeutlichten Weise durch Einschieben und Verdrehen der Austragbaugruppe 90 mit korrespondierenden Bajonettnocken 98 eine Kopplung gestattet.

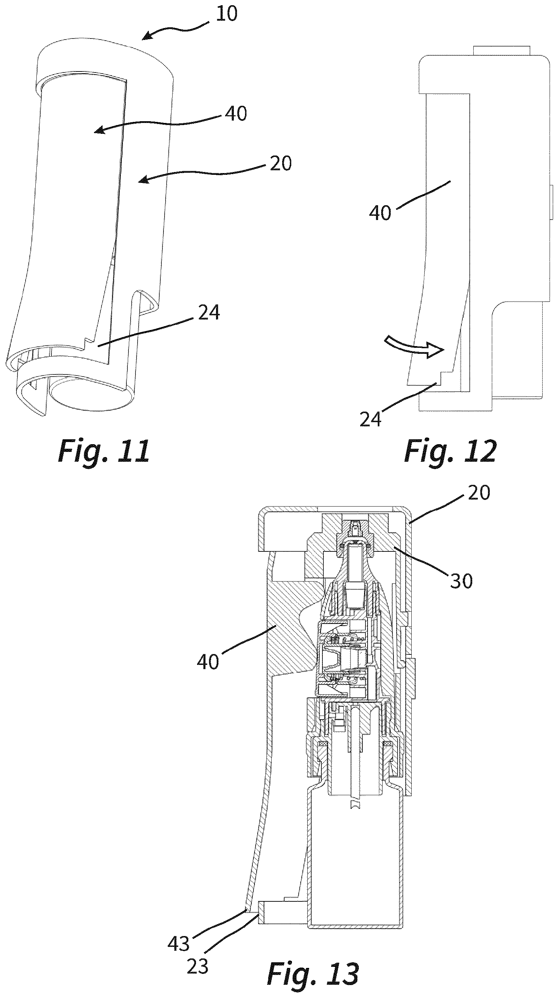

Bei der Gestaltung gemäß der Fig. 11 bis 13 ist gegenüber der Gestaltung der Fig. 1 bis 10 nur eine im Erfindungskontext wesentliche Änderung zu verzeichnen. Diese besteht darin, dass die Betätigungshandhabe 40 nicht vollständig außenseitig des Außengehäuses 20 angeordnet ist, sondern durch eine Durchbrechung 24 des Außengehäuses 20 in dieses hineinragt. Die Betätigungshandhabe 40 erstreckt sich jedoch zumindest mit ihrem distalen Ende 43 bis durch die Durchbrechung 24 hindurch. Die Fig. 11 und 12 zeigen den freigebenden Zustand des Flüssigkeitsspenders 10. Ausgehend von diesem Zustand kann die Betätigungshandhabe 40 um die Schwenkachse 8 verschwenkt werden, wobei hierdurch ein Austragvorgang bewirkt wird.

Wird allerdings das Außengehäuse 20 zur Herstellung des gesperrten und dabei vorzugsweise auch blockierten Zustandes gegenüber dem Innengehäuse 30 und der Betätigungshandhabe 40 nach oben verschoben, so stellt sich der Zustand der Fig. 13 ein. In diesem Zustand untergreift ein unterer Randbereich 23 des Außenkörpers 20 die Betätigungshandhabe 40 im Bereich ihres distalen Endes 43. Wie sich aus Fig. 3 gut ersehen lässt, ist dadurch eine Schwenkbeweglichkeit der Betätigungshandhabe 40 stark eingeschränkt. Die Betätigungshandhabe 40 kann in diesem gesperrten Zustand nicht mehr ausreichend weit verschwenkt werden, um einen Austragvorgang zu bewirken.

Die Flüssigkeitsspeicher 80, die in den Fig. 1 bis 13 gezeigt sind, sind in Form einwandiger starrer Flüssigkeitsspeicher ausgebildet, die über eine Belüftung verfügen, so dass nach dem Austritt von Flüssigkeit zum Zwecke des Druckausgleichs Luft aus der Umgebung nachströmen kann.

Demgegenüber bevorzugt sind die Flüssigkeitsspeichergestaltungen, die in den Fig. 14 bis 16 dargestellt sind. Bei der Gestaltung gemäß Fig. 14 und 15 ist der Flüssigkeitsspeicher 80 mit einer starren Außenhülle 81 versehen, innerhalb derer ein Flüssigkeitsbeutel 83 angeordnet ist. Dieser Flüssigkeitsbeutel wird durch flexible Wandungen 84 gebildet, so dass er sich im Zuge des Austrags von Flüssigkeit verkleinern kann, ohne dass es zu einem dauerhaften Unterdruck im Flüssigkeitsspeicher 80 kommt. Bei der konkreten Gestaltung der Fig. 14 und 15 ist eine Nachfüllöffnung 86 vorgesehen, durch die aus einem Nachfüllspender, wie beispielsweise einem Druckspeicher, neue Flüssigkeit in den Flüssigkeitsspeicher 80 gedrückt werden kann.

Die Ausgestaltung der Fig. 16 ist als Schleppkolbensystem ausgestaltet. Dies bedeutet, dass hier innerhalb des zylindrischen Flüssigkeitsspeichers 80 ein axial verlagerbarer Schleppkolben 82 vorgesehen ist, der im Zuge des fortgesetzten Austrags von Flüssigkeit nach oben nachgezogen wird, so dass wiederum keine Belüftung erforderlich ist und dennoch ein dauerhafter Unterdruck im Flüssigkeitsspeicher vermieden wird.

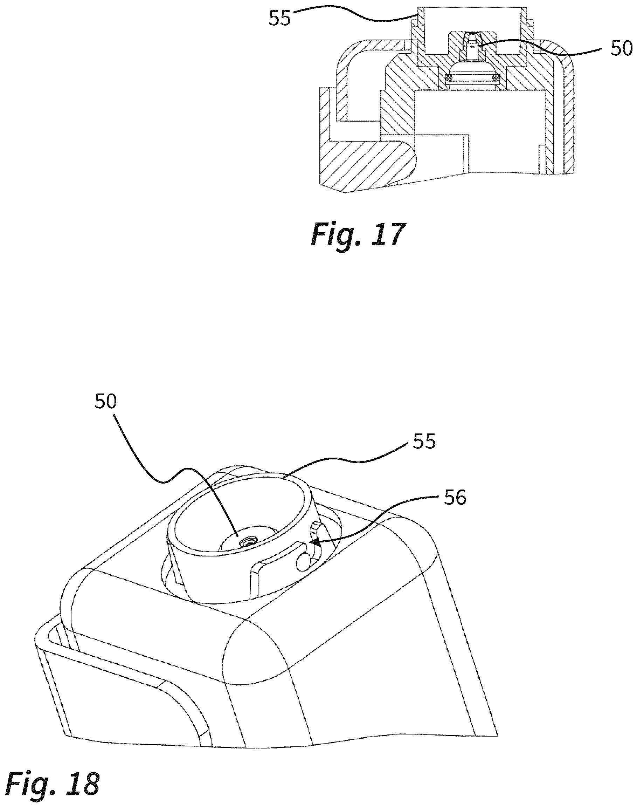

Die Fig. 17 und 18 zeigen eine besondere Ausgestaltung der Anbringung der Düseneinheit50. Im Falle der Gestaltung der Fig. 17 und 18 ist die Düseneinheit 50 nicht innerhalb eines dauerhaft mit dem Innengehäuse 30 verbundenen Trägers festgelegt, sondern in einem Wechselträger 55, der durch eine einfach lösbare Verbindung, insbesondere eine Bajonettkupplung56, mit einem Austragstutzen 52 des Innengehäuses 30 gekoppelt ist. Auf diese Art und Weise kann die Einheit umfassend den Wechselträger 55 in technischer und ästhetischer Weise an die Wünsche des Nutzers angepasst werden. Weiterhin ist eine Reinigung der Düseneinheit 50 durch einen solchen einfachen Wechselmechanismus in einfacher Weise möglich.

## Patentansprüche

1. Flüssigkeitsspender (10), insbesondere zum Austrag einer pharmazeutischen Flüssigkeit oder eine Nikotin oder Cannabis enthaltenden Flüssigkeit, mit den folgenden Merkmalen:
a. der Flüssigkeitsspender (10) weist einen Flüssigkeitsspeicher (80) zur Lagerung der Flüssigkeit vor dem Austrag auf, und
b. der Flüssigkeitsspender (10) weist eine Düseneinheit (50) auf, durch die Flüssigkeit in einer Austragrichtung (2) austragbar ist, und
c. der Flüssigkeitsspender (10) weist eine Betätigungshandhabe (40) auf, die in einer von der Austragrichtung (2) abweichenden Richtung (6) betätigbar ist, um Flüssigkeit aus dem Flüssigkeitsspeicher (80) zur Düseneinheit (50) zu fördern,
**gekennzeichnet durch** die folgenden weiteren Merkmale:
d. der Flüssigkeitsspender (10) weist ein Innengehäuse (30) auf, an dem die Betätigungshandhabe (40) schwenkbar angelenkt ist, und
e. der Flüssigkeitsspender (10) weist ein Außengehäuse (20) auf, welches gegenüber dem Innengehäuse (30) translativ zwischen einer ersten Endlage und einer zweiten Endlage verschieblich ist, wobei die Endlagen die Beweglichkeit des Außengehäuses (20) gegenüber dem Innengehäuse (30) begrenzen, und
f. die Betätigungshandhabe (40) und das Außengehäuse (20) weisen zusammenwirkende Sperrabschnitte (42, 43, 22, 23) auf, die in der ersten Endlage eine Betätigung der Betätigungshandhabe (40) unterbinden und die in der zweiten Endlage eine Betätigung der Betätigungshandhabe (40) ermöglichen.

2. Flüssigkeitsspender (10) nach Anspruch 1 mit dem folgenden weiteren Merkmal:
a. der Flüssigkeitsspender (10) verfügt über eine Blockiereinrichtung (12), mittels derer die Verschiebbarkeit des Außengehäuses (20) gegenüber dem Innengehäuse (30) blockierbar ist,
vorzugsweise mit mindestens einem der folgenden zusätzlichen Merkmale:
b. am Innengehäuse (30) oder am Außengehäuse ist ein elastisch auslenkbarer Blockierkörper (35) vorgesehen, der in einer nicht ausgelenkten Stellung in einer Blockierausnehmung (25) des Außengehäuses (20) oder des Innengehäuses angeordnet ist und hierdurch die Blockierung bewirkt und der mittels mindestens einer Freigabetaste (26) derart auslenkbar ist, dass er aus der Blockierausnehmung (25) ausgerückt wird, und/oder
c. es ist eine Durchbrechung (25) im Außengehäuse (20) vorgesehen, durch die hindurch eine Indikatorfläche (36) des Innengehäuses (30) von außen sichtbar ist, wenn sich das Innengehäuse (30) und das Außengehäuse (20) in der ersten bzw. der zweiten Endlage befinden, wobei vorzugsweise die Durchbrechung (25) des Außengehäuses gleichzeitig die Blockierausnehmung (25) darstellt.

3. Flüssigkeitsspender (10) nach Anspruch 1 oder 2 mit dem folgenden weiteren Merkmal:
a. die Düseneinheit (50) ist ortsfest zum Innengehäuse (30) angeordnet,
vorzugsweise mit dem folgenden zusätzlichen Merkmal:
b. die Düseneinheit (50) und/oder ein die Düseneinheit (50) umgebender Austragstutzen (52) ist derart angeordnet, dass die Düseneinheit (50) und/oder der Austragstutzen (52) in der ersten Endlage innerhalb des Außengehäuses (20) angeordnet ist und in der zweiten Endlage durch eine Öffnung (27) des Außengehäuses (20) nach außen verlagert ist.

4. Flüssigkeitsspender (10) nach einem der vorstehenden Ansprüche mit den folgenden weiteren Merkmalen:
a. das Außengehäuse (20) weist mindestens eine sich nach außen oder nach innen erstreckenden Sperrkontur (22) auf, vorzugsweise in Art eines Stiftes, und
b. die Betätigungshandhabe (40) weist mindestens eine in Richtung der mindestens einen Sperrkontur (22) weisende Sperrkante (42) auf, zu der benachbart ein Freibereich (46) angeordnet ist, so dass in der ersten Endlage bei Betätigung der Betätigungshandhabe (40) die Sperrkante (42) mit der Sperrkontur (22) kollidiert und in der zweiten Endlage die Sperrkontur (22) bei Betätigung der Betätigungshandhabe (40) in den Freibereich (46) gelangt.

5. Flüssigkeitsspender (10) nach einem der vorstehenden Ansprüche mit den folgenden weiteren Merkmalen:
a. das Außengehäuse (20) weist eine Durchbrechung (24) auf, durch die hindurch sich die Betätigungshandhabe (40) von einem Innenbereich des Außengehäuses (20) nach außen erstreckt, und
b. die Betätigungshandhabe (40) ist derart an die Durchbrechung (24) angepasst, dass in der ersten Endlage ein distales Ende (43) der Betätigungshandhabe (40) bei Betätigung an einem Randbereich (23) der Durchbrechung (24) anschlägt und in der zweiten Endlage das distale Ende (43) der Betätigungshandhabe (40) durch die Durchbrechung (24) in den Innenbereich des Außengehäuses (20) hineinschwenkbar ist.

6. Flüssigkeitsspender (10) nach einem der vorstehenden Ansprüche mit den folgenden weiteren Merkmalen:
a. die Düseneinheit (50) verfügt über einer Düsenplatte (54) mit einer Vielzahl von Düsenöffnungen, die zur Erzeugung eines Sprühstrahls ohne Lufteinfluss (Rayleigh-Zerstäubung) ausgebildet sind, und
b. die Betätigungshandhabe (40) ist als schwenkbewegliche Betätigungshandhabe ausgebildet und weist eine Betätigungsfläche (41) von mindesten 5 cm Länge auf, vorzugsweise von mindestens 6 cm Länge.

7. Flüssigkeitsspender (10) nach Anspruch 6 mit dem folgenden weiteren Merkmal:
a. der Flüssigkeitsspender (10) weist ein Innengehäuse (30) auf, an dem die Betätigungshandhabe (40) schwenkbar angelenkt ist.

8. Flüssigkeitsspender (10) nach Anspruch 6 oder 7 mit dem folgenden weiteren Merkmal:
a. der Flüssigkeitsspender (10) weist mindestens eines der Merkmale der Ansprüche 1 bis 5 auf.

9. Flüssigkeitsspender (10) nach einem der vorstehenden Ansprüche mit dem folgenden weiteren Merkmal:
a. der Flüssigkeitsspender (10) weist eine Pumpeinrichtung (60) auf, die mittels der Betätigungshandhabe (40) bedienbar ist.

10. Flüssigkeitsspender (10) nach Anspruch 9 mit dem folgenden weiteren Merkmal:
a. die Pumpeinrichtung (60) ist derart ausgelegt und mit der Betätigungshandhabe gekoppelt und die Düseneinheit (50) sowie ein Fluidkanal von der Pumpeinrichtung (60) zur Düseneinheit sind derart ausgelegt, dass ausgehend von einer vollständig mit Wasser befüllten Pumpkammer (62) der Pumpeinrichtung (60) und bei Betätigung der Betätigungshandhabe (40) mit einer Betätigungskraft von 20 Newton ein Austrag von mindestens eine Sekunden Dauer bewirkt wird.

11. Flüssigkeitsspender (10) nach einem der vorstehenden Ansprüche mit den folgenden weiteren Merkmalen:
a. der Flüssigkeitsspender (10) weist eine Austragbaugruppe (90) auf, die mindestens den Flüssigkeitsspeicher (80), die Pumpeinrichtung (60) oder ein Auslassventil sowie eine Abgabeöffnung (92) aufweist, und
b. das Innengehäuse (30) ist mit einem Aufnahmeraum für eine Aufnahme der Austragbaugruppe (90) durch werkzeuglose Kopplung versehen,
vorzugsweise mit mindestens einem der folgenden zusätzlichen Merkmale:
c. das Innengehäuse (30) und die Austragbaugruppe (90) weisen eine Bajonettkupplungseinrichtung (94) auf, und/oder
d. das Innengehäuse und die Austragbaugruppe weisen eine Klemmkopplungseinrichtung oder eine Gewindekopplungseinrichtung auf.

12. Flüssigkeitsspender (10) nach einem der vorstehenden Ansprüche mit dem folgenden weiteren Merkmal:
a. der Flüssigkeitsspeicher (80) ist als druckloser Flüssigkeitsspeicher ausgebildet,
insbesondere mit mindestens einem der folgenden zusätzlichen Merkmale:
b. der Flüssigkeitsspeicher (80) ist als Flüssigkeitsspeicher mit veränderlichem Innenvolumen ausgebildet, insbesondere als Schleppkolbenspeicher mit einem Schleppkolben (82) oder als Beutelspeicher mit mindestens einer auslenkbaren flexiblen Wandung (84), und/oder
c. der Flüssigkeitsspender (10) weist eine von der Düseneinheit (50) getrennte Nachfüllöffnung (86) auf, wobei insbesondere vorzugsweise die Nachfüllöffnung (86) unmittelbar in einer Wandung des Flüssigkeitsspeichers (80) angeordnet ist.

13. Flüssigkeitsspender (10) nach einem der vorstehenden Ansprüche mit dem folgenden weiteren Merkmal:
a. die Düseneinheit (50) ist als werkzeuglos wechselbare Düseneinheit (50) ausgebildet,
insbesondere mit dem folgenden zusätzlichen Merkmal:
b. die Düseneinheit (50) ist mittels einer Bajonettkupplung (56) mit dem Innengehäuse (30) des Flüssigkeitsspenders (10) verbunden.

14. Flüssigkeitsspender (10) nach einem der vorstehenden Ansprüche mit einem der folgenden weiteren Merkmale:
a. der Flüssigkeitsspeicher (80) ist mit einer pharmazeutischen Flüssigkeit befüllt, oder
b. der Flüssigkeitsspeicher (80) ist mit einer nikotinhaltigen und/oder cannabishaltigen Flüssigkeit befüllt.

15. Flüssigkeitsspender (10) nach einem der vorstehenden Ansprüche mit einem der folgenden weiteren Merkmale:
a. der Flüssigkeitsspeicher (80) weist ein Innenvolumen von 50 ml oder weniger auf, vorzugsweise von 10 ml oder weniger, und/oder
b. die Pumpeinrichtung ist für eine Austragmenge von mindestens 25 µl je Betätigung ausgelegt, insbesondere für eine Austragmenge von mindestens 40 µl je Betätigung, wobei die Austragmenge je Betätigung vorzugsweise weniger als 200 µl beträgt, und/oder
c. ein distales Ende (43) der Betätigungshandhabe (40) ist von einer Schwenkachse (8) der Betätigungshandhabe (40) mindestens 5 cm entfernt, insbesondere mindestens 6 cm.

## Claims

1. Liquid dispenser (10), in particular for delivering a pharmaceutical fluid or a fluid containing nicotine or cannabis, having the following features:
a. the liquid dispenser (10) has a liquid reservoir (80) for storing the liquid prior to delivery; and
b. the liquid dispenser (10) has a nozzle unit (50) by way of which the liquid is able to be delivered in a delivery direction (2), and
c. the liquid dispenser (10) has an activation handle (40) which is able to be activated in a direction (6) deviating from the delivery direction (2) in order to convey fluid from the liquid reservoir (80) to the nozzle unit (50),
**characterized by** the following further features:
d. the liquid dispenser (10) has an inner housing (30) to which the activation handle (40) is pivotably articulated, and
e. the liquid dispenser (10) has an outer housing (20), which is displaceable in a translatory manner between a first end position and a second end position relative to the inner housing (30), wherein the end positions delimit the mobility of the outer housing (20) relative to the inner housing (30), and
f. the activation handle (40) and the outer housing (20) have interacting blocking portions (42, 43, 22, 23), which prevent activation of the activation handle (40) in the first end position, and which enable activation of the activation handle (40) in the second end position.

2. Liquid dispenser (10) according to Claim 1, having the following further feature:
a. the liquid dispenser (10) has a blocking device (12) by means of which the displacement capability of the outer housing (20) relative to the inner housing (30) is able to be blocked,
preferably having at least one of the following additional features:
b. provided on the inner housing (30) or on the outer housing is an elastically deflectable blocking member (35) which in a non-deflected position is disposed in a blocking recess (25) of the outer housing (20) or of the inner housing and thereby causes blocking, and which can be deflected by means of at least one release button (26) in such a manner that said blocking member (35) is disengaged from the blocking recess (25), and/or
c. provided in the outer housing (20) is a cut-out (25) through which an indicator surface (36) of the inner housing (30) is visible from the outside when the inner housing (30) and the outer housing (20) are in the first or the second end position, whereby preferably the cut-out (25) of the outer housing simultaneously represents the blocking recess (25).

3. Liquid dispenser (10) according to Claim 1 or 2, having the following further feature:
a. the nozzle unit (50) is disposed so as to be stationary relative to the inner housing (30),
preferably comprising the following additional feature:
b. the nozzle unit (50), and/or a delivery port (52) surrounding the nozzle unit (50), are/is disposed in such a way that the nozzle unit (50) and/or the delivery port (52) are/is disposed in the first end position within the outer housing (20) and in the second end position are/is displaced outwards through an opening (27) of the outer housing (20).

4. Liquid dispenser (10) according to one of the preceding claims, having the following further features:
a. the outer housing (20) has at least one outwardly or inwardly extending blocking contour (22), preferably in the manner of a pin, and
b. the activation handle (40) has at least one blocking edge (42) which points in the direction of the at least one blocking contour (22) and adjacent to which a void (46) is disposed in such a way that in the first end position, when the activation handle (40) is activated, the blocking edge (42) collides with the blocking contour (22), and in the second end position, when the activation handle (40) is activated, the blocking contour (22) enters the void (46).

5. Liquid dispenser (10) according to one of the preceding claims, having the following further features:
a. the outer housing (20) has a cut-out (24) through which the activation handle (40) extends outward from an inner region of the outer housing (20), and
b. the activation handle (40) is adapted to the cut-out (24) in such a manner that, in the first end position, a distal end (43) of the activation handle (40) stops at an edge region (23) of the cut-out (24) during actuation and, in the second end position, the distal end (43) of the activation handle (40) is pivotable into the inner region of the outer housing (20) by way of the cut-out (24).

6. Liquid dispenser (10) according to one of the preceding claims, having the following further features:
a. the nozzle unit (50) has a nozzle plate (54) having a multiplicity of nozzle openings which are designed for generating a spray jet without air influence (Rayleigh atomization), and
b. the activation handle (40) is designed as a pivotable activation handle and has an activation surface (41) of at least 5 cm in length, preferably of at least 6 cm in length.

7. Liquid dispenser (10) according to Claim 6, having the following further feature:
a. the liquid dispenser (10) has an inner housing (30) on which the operating handle (40) is pivotally articulated.

8. Liquid dispenser (10) according to Claim 6 or 7, having the following further feature:
a. the liquid dispenser (10) has at least one of the features of Claims 1 to 5.

9. Liquid dispenser (10) according to one of the preceding claims, having the following further feature:
a. the liquid dispenser (10) has a pump device (60) which is able to be operated by means of the activation handle (40).

10. Liquid dispenser (10) according to Claim 9, having the following further feature:
a. the pump device (60) is conceived and coupled to the activation handle in such a manner, and the nozzle unit (50) and a fluid channel from the pump device (60) to the nozzle unit are conceived in such a manner, that proceeding from a pump chamber (62) of the pump device (60) completely filled with water, and when the activation handle (40) is actuated with an actuating force of 20 newtons, a delivery with a duration of at least one second is effected.

11. Liquid dispenser (10) according to one of the preceding claims, having the following further features:
a. the liquid dispenser (10) has a delivery module (90) which comprises at least the liquid reservoir (80), the pump device (60) or an outlet valve and a discharge port (92), and
b. the inner housing (30) is provided with a receptacle space for receiving the delivery module (90) by means of a tool-free coupling,
preferably having at least one of the following additional features:
c. the inner housing (30) and the delivery module (90) have a bayonet coupling device (94), and/or
d. the inner housing and the delivery module have a clamping coupling device or a threaded coupling device.

12. Liquid dispenser (10) according to one of the preceding claims, having the following further feature:
a. the liquid reservoir (80) is designed as a non-pressurized liquid reservoir,
preferably having at least one of the following additional features:
b. the liquid reservoir (80) is designed as a liquid reservoir with a variable internal volume, in particular as a drag piston reservoir with a drag piston (82) or as a bag reservoir with at least one deflectable flexible wall (84), and/or
c. the liquid dispenser (10) has a refill opening (86) separate from the nozzle unit (50), whereby particularly preferably the refill opening (86) is disposed directly in a wall of the liquid reservoir (80).

13. Liquid dispenser (10) according to one of the preceding claims, having the following further feature:
a. the nozzle unit (50) is designed as a nozzle unit (50) which is replaceable without tools,
in particular having the following additional feature:
b. the nozzle unit (50) is connected to the inner housing (30) of the liquid dispenser (10) by means of a bayonet coupling (56).

14. Liquid dispenser (10) according to one of the preceding claims, having one of the following further features:
a. the liquid reservoir (80) is filled with a pharmaceutical liquid, or
b. the liquid reservoir (80) is filled with a nicotine-containing and/or cannabis-containing fluid.

15. Liquid dispenser (10) according to one of the preceding claims, having one of the following further features:
a. the liquid reservoir (80) has an internal volume of 50 ml or less, preferably of 10 ml or less, and/or
b. The pump device is designed for a delivery rate of at least 25 µl per activation, in particular for a delivery rate of at least 40 µl per activation, wherein the delivery rate per activation is preferably less than 200 µl, and/or
c. a distal end (43) of the activation handle (40) is at least 5 cm, in particular at least 6 cm, away from a pivot axis (8) of the activation handle (40).

## Revendications

1. Distributeur de liquide (10), notamment pour distribuer un liquide pharmaceutique ou un liquide contenant de la nicotine ou du cannabis, avec les caractéristiques suivantes :
a. le distributeur de liquide (10) présente un réservoir de liquide (80) pour stocker le liquide avant la distribution, et
b. le distributeur de liquide (10) présente une unité de buse (50) à travers laquelle le liquide peut être distribué dans une direction de distribution (2), et
c. le distributeur de liquide (10) présente une poignée d'actionnement (40) qui peut être actionnée dans une direction (6) différente de la direction de distribution (2) afin de transporter du liquide depuis le réservoir de liquide (80) vers l'unité de buse (50),
**caractérisé par** les caractéristiques supplémentaires suivantes :
d. le distributeur de liquide (10) présente un boîtier intérieur (30) sur lequel la poignée d'actionnement (40) est articulée de manière pivotante, et
e. le distributeur de liquide (10) présente un boîtier extérieur (20) qui peut être déplacé en translation par rapport au boîtier intérieur (30) entre une première position d'extrémité et une deuxième position d'extrémité, les positions d'extrémité limitant la mobilité du boîtier extérieur (20) par rapport au boîtier intérieur (30), et
f. la poignée d'actionnement (40) et le boîtier extérieur (20) présentent des sections d'arrêt (42, 43, 22, 23) coopérantes, qui empêchent un actionnement de la poignée d'actionnement (40) dans la première position d'extrémité et qui permettent un actionnement de la poignée d'actionnement (40) dans la deuxième position d'extrémité.

2. Distributeur de liquide (10) selon la revendication 1, avec la caractéristique supplémentaire suivante :
a. le distributeur de liquide (10) dispose d'un dispositif de blocage (12) au moyen duquel la capacité de déplacement du boîtier extérieur (20) par rapport au boîtier intérieur (30) peut être bloquée,
de préférence avec au moins l'une quelconque des caractéristiques supplémentaires suivantes :
b. il est prévu sur le boîtier intérieur (30) ou sur le boîtier extérieur un corps de blocage (35) pouvant être dévié élastiquement, qui est agencé dans une position non déviée dans un évidement de blocage (25) du boîtier extérieur (20) ou du boîtier intérieur et qui provoque ainsi le blocage et qui peut être dévié au moyen d'au moins une touche de libération (26) de telle sorte qu'il est dégagé de l'évidement de blocage (25), et/ou
c. il est prévu un ajour (25) dans le boîtier extérieur (20), à travers lequel une surface indicatrice (36) du boîtier intérieur (30) est visible de l'extérieur lorsque le boîtier intérieur (30) et le boîtier extérieur (20) se trouvent respectivement dans la première et la deuxième position d'extrémité, l'ajour (25) du boîtier extérieur constituant de préférence en même temps l'évidement de blocage (25).

3. Distributeur de liquide (10) selon la revendication 1 ou 2, avec la caractéristique supplémentaire suivante :
a. l'unité de buse (50) est agencée de manière fixe par rapport au boîtier intérieur (30),
de préférence avec la caractéristique supplémentaire suivante :
b. l'unité de buse (50) et/ou une tubulure de distribution (52) entourant l'unité de buse (50) est agencée de telle sorte que l'unité de buse (50) et/ou la tubulure de distribution (52) est agencée à l'intérieur du boîtier extérieur (20) dans la première position d'extrémité et est déplacée vers l'extérieur à travers une ouverture (27) du boîtier extérieur (20) dans la deuxième position d'extrémité.

4. Distributeur de liquide (10) selon l'une quelconque des revendications précédentes, avec les caractéristiques supplémentaires suivantes :
a. le boîtier extérieur (20) présente au moins un contour d'arrêt (22) s'étendant vers l'extérieur ou vers l'intérieur, de préférence à la manière d'une broche, et
b. la poignée d'actionnement (40) présente au moins un bord d'arrêt (42) orienté en direction de l'au moins un contour d'arrêt (22), au voisinage duquel est agencée une zone libre (46), de telle sorte que dans la première position d'extrémité, lors de l'actionnement de la poignée d'actionnement (40), le bord d'arrêt (42) entre en collision avec le contour d'arrêt (22) et, dans la deuxième position d'extrémité, le contour d'arrêt (22) parvient dans la zone libre (46) lors de l'actionnement de la poignée d'actionnement (40).

5. Distributeur de liquide (10) selon l'une quelconque des revendications précédentes, avec les caractéristiques supplémentaires suivantes :
a. le boîtier extérieur (20) présente un ajour (24) à travers lequel la poignée d'actionnement (40) s'étend vers l'extérieur depuis une zone intérieure du boîtier extérieur (20), et
b. la poignée d'actionnement (40) est adaptée à l'ajour (24) de telle sorte que, dans la première position d'extrémité, une extrémité distale (43) de la poignée d'actionnement (40) vient buter contre une zone de bord (23) de l'ajour (24) lors de l'actionnement et, dans la deuxième position d'extrémité, l'extrémité distale (43) de la poignée d'actionnement (40) peut pivoter à travers l'ajour (24) dans la zone intérieure du boîtier extérieur (20).

6. Distributeur de liquide (10) selon l'une quelconque des revendications précédentes, avec les caractéristiques supplémentaires suivantes :
a. l'unité de buse (50) dispose d'une plaque de buse (54) ayant une pluralité d'ouvertures de buse configurées pour produire un jet de pulvérisation sans air (pulvérisation Rayleigh), et
b. la poignée d'actionnement (40) est configurée sous forme de poignée d'actionnement mobile en pivotement et présente une surface d'actionnement (41) d'au moins 5 cm de long, de préférence d'au moins 6 cm de long.

7. Distributeur de liquide (10) selon la revendication 6, avec la caractéristique supplémentaire suivante :
a. le distributeur de liquide (10) présente un boîtier intérieur (30) sur lequel la poignée d'actionnement (40) est articulée de manière pivotante.

8. Distributeur de liquide (10) selon la revendication 6 ou 7, avec la caractéristique supplémentaire suivante :
a. le distributeur de liquide (10) présente au moins l'une quelconque des caractéristiques des revendications 1 à 5.

9. Distributeur de liquide (10) selon l'une quelconque des revendications précédentes, avec la caractéristique supplémentaire suivante :
a. le distributeur de liquide (10) présente un dispositif de pompage (60) qui peut être commandé au moyen de la poignée d'actionnement (40).

10. Distributeur de liquide (10) selon la revendication 9, avec la caractéristique supplémentaire suivante :
a. le dispositif de pompage (60) est conçu et couplé à la poignée d'actionnement et l'unité de buse (50) ainsi qu'un canal de fluide allant du dispositif de pompage (60) à l'unité de buse sont conçus de telle sorte qu'une distribution d'une durée d'au moins une seconde est effectuée à partir d'une chambre de pompage (62) du dispositif de pompage (60) entièrement remplie d'eau et lors de l'actionnement de la poignée d'actionnement (40) avec une force d'actionnement de 20 Newtons.

11. Distributeur de liquide (10) selon l'une quelconque des revendications précédentes, avec les caractéristiques supplémentaires suivantes :
a. le distributeur de liquide (10) présente un ensemble de distribution (90) présentant au moins le réservoir de liquide (80), le dispositif de pompage (60) ou une soupape de sortie et une ouverture de déchargement (92), et
b. le boîtier intérieur (30) est pourvu d'un espace de réception pour recevoir l'ensemble de distribution (90) par couplage sans outil,
de préférence avec au moins l'une quelconque des caractéristiques supplémentaires suivantes :
c. le boîtier intérieur (30) et l'ensemble de distribution (90) présentent un dispositif d'accouplement à baïonnette (94), et/ou
d. le boîtier intérieur et l'ensemble de distribution présentent un dispositif d'accouplement par serrage ou un dispositif d'accouplement par filetage.

12. Distributeur de liquide (10) selon l'une quelconque des revendications précédentes, avec la caractéristique supplémentaire suivante :
a. le réservoir de liquide (80) est configuré sous forme de réservoir de liquide sans pression,
notamment avec au moins l'une quelconque des caractéristiques supplémentaires suivantes :
b. le réservoir de liquide (80) est configuré sous forme de réservoir de liquide à volume intérieur variable, notamment sous forme de réservoir à piston traînant avec un piston traînant (82) ou sous forme de réservoir à poche avec au moins une paroi flexible (84) pouvant être déviée, et/ou
c. le distributeur de liquide (10) présente une ouverture de remplissage (86) séparée de l'unité de buse (50), l'ouverture de remplissage (86) étant de préférence agencée directement dans une paroi du réservoir de liquide (80).

13. Distributeur de liquide (10) selon l'une quelconque des revendications précédentes, avec la caractéristique supplémentaire suivante :
a. l'unité de buse (50) est configurée sous forme d'unité de buse (50) remplaçable sans outil,
notamment avec la caractéristique supplémentaire suivante :
b. l'unité de buse (50) est reliée au boîtier intérieur (30) du distributeur de liquide (10) au moyen d'un accouplement à baïonnette (56).

14. Distributeur de liquide (10) selon l'une quelconque des revendications précédentes, avec l'une quelconque des caractéristiques supplémentaires suivantes :
a. le distributeur de liquide (80) est rempli d'un liquide pharmaceutique, ou
b. le réservoir de liquide (80) est rempli d'un liquide contenant de la nicotine et/ou contenant du cannabis.

15. Distributeur de liquide (10) selon l'une quelconque des revendications précédentes, avec l'une quelconque des caractéristiques supplémentaires suivantes :
a. le réservoir de liquide (80) présente un volume intérieur de 50 ml ou moins, de préférence de 10 ml ou moins, et/ou
b. le dispositif de pompage est conçu pour un débit de distribution d'au moins 25 µl par actionnement, notamment pour un débit de distribution d'au moins 40 µl par actionnement, le débit de distribution par actionnement étant de préférence inférieur à 200 µl, et/ou
c. une extrémité distale (43) de la poignée d'actionnement (40) est éloignée d'au moins 5 cm, notamment d'au moins 6 cm, d'un axe de pivotement (8) de la poignée d'actionnement (40).
